# EUROPÄISCHE PATENTSCHRIFT

(11) **EP 0 542 079 B1**
(45) Veröffentlichungstag und Bekanntmachung des Hinweises auf die Patenterteilung: **23.07.1997**
(21) Anmeldenummer: 92118736.5
(22) Anmeldetag: 02.11.1992
(51) Int. Cl.: C09B 62/20, C07D 239/42

(54) **Verfahren zur kontinuierlichen Umsetzung von Halogenpyrimidinen mit Aminen**
Process for continuously reacting halogenopyrimidines with amines
Méthode de réaction en continu d'halogénopyrimidines avec des amines

(30) Priorität: 13.11.1991 DE 4137291
(43) Veröffentlichungstag der Anmeldung: 19.05.1993
(73) Patentinhaber: BAYER AG, 51368 Leverkusen (DE)
(72) Erfinder: Arnold, Siegbert, Dr., W-5300 Bonn (DE); Frosch, Hans-Georg, Dr., W-5000 Köln 91 (DE); Hoppe, Manfred, Dr., W-5067 Kürten 2 (DE); Müllers, Wolfgang, Dr., W-5060 Bergisch Gladbach 1 (DE); Sommer, Richard, Dr., W-5068 Odenthal-Glöbusch (DE)

(56) Entgegenhaltungen:
- EP-A- 0 043 927
- EP-A- 0 458 110
- EP-A- 0 494 456

## Beschreibung

Die Anmeldung betrifft ein Verfahren zur kontinuierlichen Umsetzung von Halogenpyrimidinen mit Aminen.

Die Umsetzungen von Halogenpyrimidinen, wie sie beispielsweise aus DE-A 1 644 204 bekannt sind, mit Aminen werden üblicherweise im Rührwerksbehälter so durchgeführt, daß die Amin-Lösung bzw. -Suspension vorgelegt und das Halogenpyrimidin unter definierten Bedingungen zudosiert wird. Nachteilig bei dieser Vorgehensweise ist unter anderem, daß die Halogenpyrimidine in dem wäßrigen Medium nicht löslich sind, so daß in dem resultierenden Zweiphasensystem neben der gewünschten Umsetzung des Halogenpyrimidins mit der Aminogruppe in teilweise erheblichem Maße Hydrolyse des bzw. der reaktivsten Halogenatoms bzw. -atome eintritt. Dies hat zur Folge, daß teilweise erhebliche Halogenpyrimidin-Überschüsse zur vollständigen Umsetzung des Amins erforderlich sind.

Ein weiterer Nachteil der derzeit üblichen Umsetzung in Rührwerksbehältern besteht darin, daß vor allem in den Fällen, bei denen besonders reaktionsfähige Amine und/oder besonders reaktionsfähige Halogenpyrimidine miteinander umgesetzt werden, die Reaktion nicht bei der gewünschten einfachen Umsetzung stehen bleibt, sondern daß z.T. in erheblichem Ausmaß zwei Amin-Moleküle mit dem Halogenpyrimidin reagieren.

Es besteht die Aufgabe, ein verbessertes Verfahren für die Umsetzung von Halogenpyrimidinen mit Aminen, insbesondere Aminonaphtholsulfonsäuren zu finden.

Die vorliegende Erfindung betrifft ein Verfahren zur kontinuierlichen Umsetzung von Halogenpyrimidinen mit Aminen, wobei Halogenpyrimidin und eine wäßrige Aminlösung in einen Reaktor eingeleitet werden und das Reaktionsprodukt anschließend abgeleitet wird, dadurch gekennzeichnet, daß die Edukte gleichzeitig und kontinuierlich in den Reaktor eingeleitet werden unter intensiver Vermischung.

Bei der Durchführung des erfindungsgemäßen Verfahrens werden die Halogenpyrimidine in fein dispergierter Form mit dem Amin zur Umsetzung gebracht, wodurch die Reaktionsgeschwindigkeiten erheblich erhöht werden. Dabei können die Reaktionsbedingungen, vor allem Temperatur und pH-Wert, so gewählt werden, daß die Reaktionsgeschwindigkeit noch stärker erhöht wird, ohne daß die Hydrolyse des Halogenpyrimidins oder des Reaktionsproduktes in stärkerem Maße auftritt.

Ein weiterer Vorteil ist, daß bei reaktionsfähigen Aminen und/oder Halogenpyrimidinen Mehrfachreaktionen durch die exakt einzustellende Stöchiometrie in der Reaktionszone vermieden, zumindest aber stark zurückgedrängt werden können.

Man kann die Halogenpyrimidine in größerem Überschuß verwenden, zweckmäßig sind jedoch Halogenpyrimidin/Amin Molverhältnisse von 0,8:1,0 bis 1,5:1, vorzugsweise 1:1 bis 1,2:1, insbesondere 1:1 bis 1,02:1 einzusetzen.

Geeignete Reaktoren sind solche, bei denen die Reaktionspartner im stöchiometrisch gewünschten Verhältnis unter hohem Energieeintrag unter Vermeidung, zumindest aber Minimierung von Rückvermischung intensiv miteinander vermischt werden, wobei die Reaktionsbedingungen z.B. Temperatur und pH-Wert so gewählt werden, daß in dem Reaktor bereits ein erheblicher Umsatz erfolgt.

Geeignete Reaktoren sind beispielsweise Düsenreaktoren wie bei Zehner, P. u. Bittins, K.: Fortschr. Verf. Technik D 23, 1985, S. 373-393 beschrieben, bei denen die Edukte gleichzeitig und kontinuierlich mit unterschiedlichen Geschwindigkeiten in den Reaktor eingeleitet werden und durch die Differenz der geschwindigkeiten eine intensive Vermischung bewirkt wird, und dabei die Umsetzung bei weitgehend rückver mischunsfreier Strömung zu einem erheblichen Teil in diesem Reaktor abgeschlossen wird.

Gemäß einer besonderen Durchführungsform des neuen Verfahrens strömt das Halogenpyrimidin mit einer Reynoldszahl von mindestens 10 000, vorzugsweise mindestens 15 000, und die wäßrige Aminlösung mit einer solchen von mindestens 2 500, vorzugsweise mindestens 5 000, in den Reaktor ein, wobei die Differenz der Strömungsgeschwindigkeiten zwischen Halogenpyrimidinstrom und Aminlösungsstrom mindestens 20 m/s, vorzugsweise mindestens 40 m/s beträgt.

Diese Maßnahmen gewährleisten eine besonders intensive Vermischung auf kürzestem Wege ohne Rückströmung.

Vorzugsweise wird ein Verhältnis des Reaktorquerschnittes zum Einströmquerschnitt des Halogenpyrimidinstromes von 225 bis 40 000, vorzugsweise von 700 bis 12 000, eingehalten.

Durch diese Anpassung des Querschnittsverhältnisses an das Massenstromverhältnis wird die rückströmungsfreie Durchmischung der Reaktionspartner optimiert.

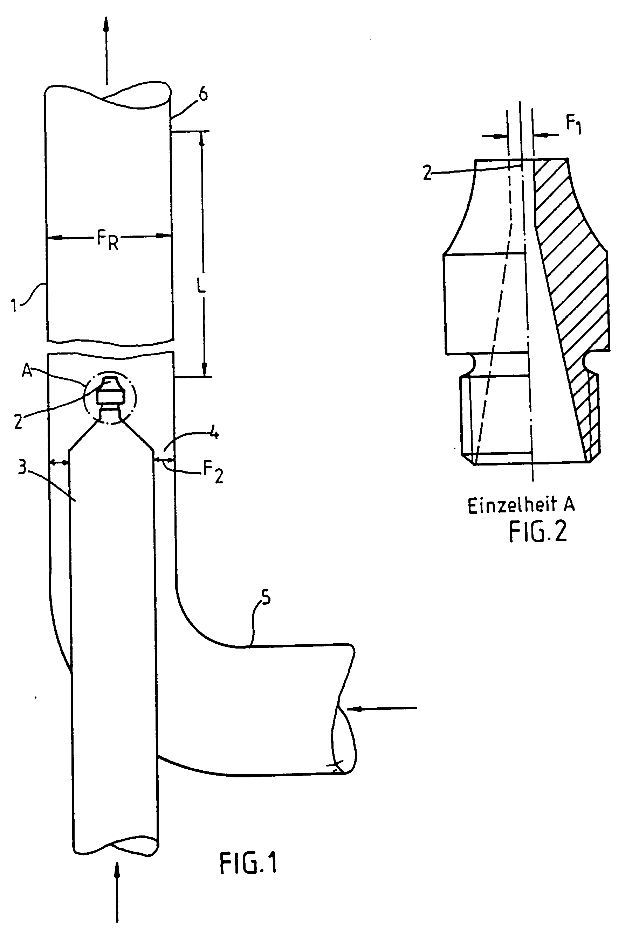

Der bevorzugte Reaktor ist in der Zeichnung Abb. 1 schematisch dargestellt und nachstehend näher erläutert. Es zeigen:
- Fig. 1: den Reaktor im Schnitt und
- Fig. 2: die Düse gemäß Einzelheit A zur Hälfte im Schnitt in vergrößerter Darstellung.

In ein Reaktionsrohr 1 von F_{R} = 80 mm² Kreisquerschnitt mündet axial über eine Düse 2 von F₁ = 0,03 mm² Querschnitt eine Zuleitung für das Halogenpyrimidin. Sie ist konzentrisch von einer Ringdüse 4 von F₂ = 64 mm² Querschnitt umgeben, welche mit einer Zuleitung 5 für eine wäßrige Aminlösung verbunden ist, Die Länge der Reaktionszone L beträgt etwa 250 mm; der anschließende abschnitt 6 dient als Transportleitung für das Reaktionsprodukt.

Anstelle einer Ringdüse 4 können auch mehrere einzelne Düsen bzw. Düsenöffnungen über den Umfang verteilt sein.

Weitere geeignete Reaktoren sind Dispergiereinheiten des Typs Rotor/Stator-Mischer.

Die Verweilzeit in den Reaktoren reicht aus, um einen erheblichen Umsetzungsgrad bereits im Reaktor zu gewährleisten.

Zur Beendigung der Reaktion können weitere kontinuierlich durchströmte Reaktoren wie z.B. Rotor/Stator-Systeme, Strömungsrohr evtl. mit Statikmischer und werksbehälter verwendet werden. Alternativ kann die Reaktion auch in diskontinierlich betriebenen Rührwerksbehältern zu Ende geführt werden.

Gemäß einer weiteren besonders bevorzugten Durchführungsform des neuen Verfahrens wird die Reaktion bei Temperaturen von 0 bis 90°C, vorzugsweise 0 bis 50°C durchgeführt.

Die Alkalimenge in der Aminlösung wird so gewählt, daß sich gegen Ende der Reaktion ein pH-Wert zwischen 1 und 11, vorzugsweise zwischen 3 und 9, einstellt.

Vorzugsweise setzt man bei Lösungen von Aminen, bei denen die Aminogruppe am aromatischen Kern direkt gebunden ist, eine Puffersubstanz zu, die bewirkt, daß während der Umsetzung, je nach Puffersubstanz, ein pH-Wert zwischen 1 und 8, vorzugsweise zwischen 2 und 5, beibehalten bleibt. Geeignete Puffersubstanzen sind z.B. Alkalimetallfluoride oder Alkalimetallphosphate, insbesondere NaF, Na₂HPO₄, Na₃PO₄ bzw. Gemische hieraus. Diese Puffersubstanzen werden im allgemeinen in einer Menge von 0,2 bis 2, vorzugsweise 0,4 bis 1,2 Mol je Mol Amin eingesetzt.

Wird ein chromophores Amin mit einem Halogenpyrimidin umgesetzt, kann der erhaltene Reaktivfarbstoff isoliert oder ohne Zwischenisolierung direkt getrocknet werden.

Bei Umsetzung eines Halogenpyrimidins mit einem nichtchromophoren Amin kann das Reaktionsprodukt isoliert werden, es wird jedoch vorzugsweise ohne Zwischenisolierung weiterverarbeitet, z.B. zu Reaktivfarbstoffen, entweder durch nachfolgende Diazotierung und Kupplung mit einer Kupplungskomponente oder durch Umsatz mit einem Diazoniumsalz eines aromatischen Amins.

Diese Weiterverarbeitung kann diskontinuierlich oder kontinuierlich auf bekannte Art und Weise durchgeführt werden.

Nach dem erfindungsgemäßen Verfahren erhält man die Kondensationsprodukte aus den Halogenpyrimidinen und Aminen so in vielen Fällen in deutlich höherer Reinheit und in höheren Ausbeuten als nach den bisher üblichen Verfahren. Dies wirkt sich positiv auf die Qualität der aus den Kondensationsprodukten hergestellten Reaktivfarbstoffen aus.

Vorzugsweise werden Halogenpyrimidine Hal-Z kontinuierlich in speziellen Reaktoren mit Aminen, bevorzugt mit sulfonsäure-gruppen-haltigen der Formel 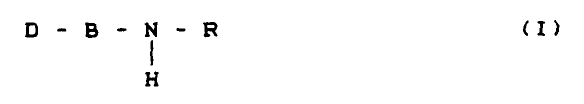 zu Reaktivstoffen bzw. Reaktivfarbstoff-Vorprodukten der Formel 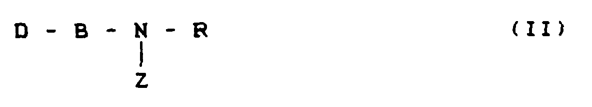 umgesetzt, worin
- B: für direkte Bindung oder Brückenglied an ein aromatisch-carbocyclisches C-Atom des Restes D,
- R: für H, gegebenenfalls substituiertes C₁-C₄-Alkyl (Substituenten vorzugsweise OH, SO₃H, OSO₃H, Cl, COOH), gegebenenfalls substituiertes Phenyl (Substituenten vorzugsweise -SO₃H, -COOH, C₁-C₄-Alkyl, Halogen), vorzugsweise jedoch für H,
- D: für einen Rest der Benzol-, Naphthalin- oder heterocyclischen Reihe, insbesondere für sulfonsäuregruppenhaltige Reste von aromatischen Aminen, die als Diazo- bzw. Kupplungskomponenten verwendet werden oder für den Rest eines Chromophors, insbesondere für den Rest eines Farbstoffs der Mono- oder Polyazo-, Metallkomplexazo-, Anthrachinon-, Phthalocyanin-, Formazan, Azomethin-, Dioxazin-, Phenazin-, Xanthen-, Thioxanthon, Naphthochinon-, Stilben- oder Triphenylmethan-Reihe steht,
- Z: für Mono-, Di- und Trihalogenpyrimidinylreste,
- Hal: für -F, -Cl und -Br steht.

Vorzugsweise wird die Reaktion in wäßrigem Medium durchgeführt unter Verwendung einer wäßrigen Amin-Lösung bzw. Amin-Suspension.

Die sulfogruppenhaltigen Amine werden als wäßrige Lösungen bzw. Suspensionen in den Reaktor eingeführt, die logenpyrimidine als Lösungen in einem inerten Lösungsmittel, vorzugsweise jedoch ohne Lösungsmittel.

Geeignete Reste D bzw. DB sind beispielsweise:

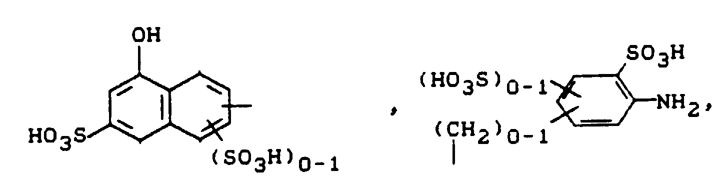

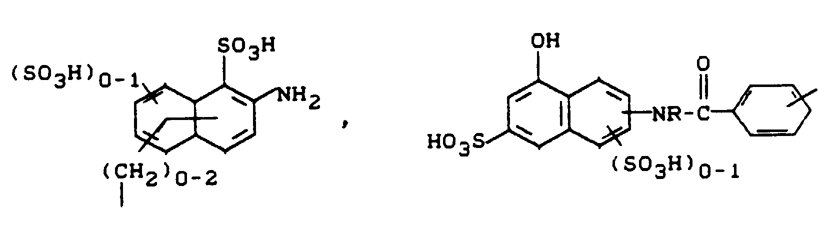

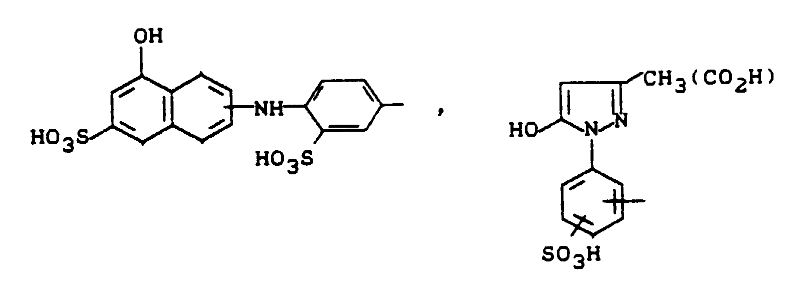

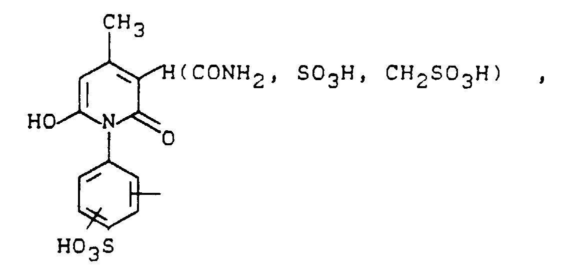

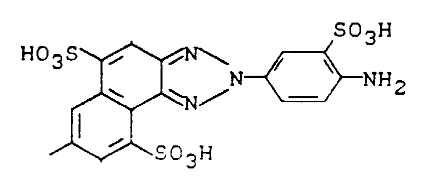

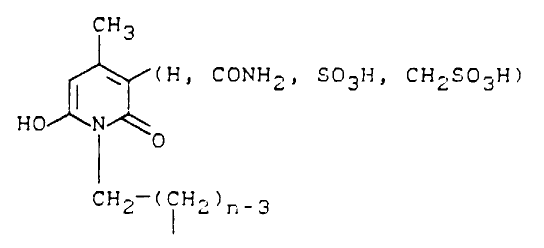

Geeignete Brückenglieder B sind beispielsweise 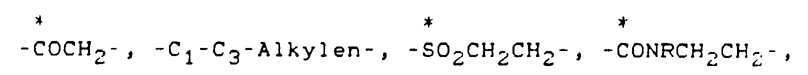 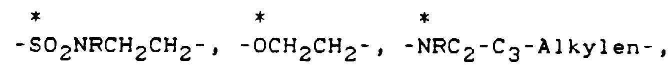 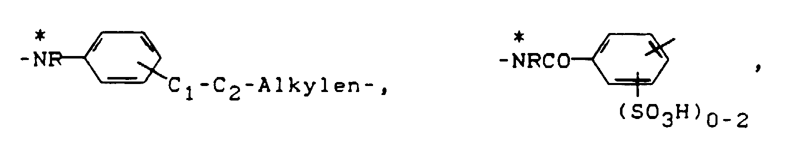 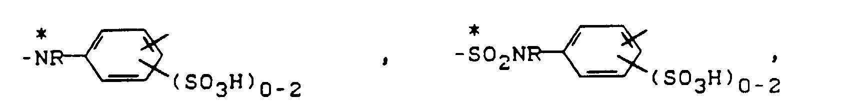 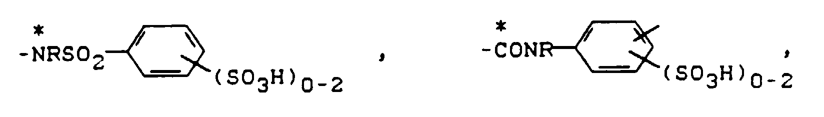 wobei der Stern die Verknüpfungsstelle mit D bezeichnet.

Geeignete sulfogruppenhaltige aromatische Amine (I) sind beispielsweise 1,4-Diaminobenzol-2,5-disulfonsäure, 1,3-Diaminobenzol-4-sulfonsäure, 1,4-Diaminobenzol-2-sulfonsäure, 1,3-Diaminobenzol-4,6-disulfonsäure, 2,5-Diaminobenzol-1,3-disulfonsäure, 1-Amino-5-hydroxynaphthalin-7-sulfonsäure, 1-Amino-8-hydroxynaphthalin-4-sulfonsäure, 1-Amino-8-hydroxynaphthalin-3-sulfonsäure, 1-Amino-8-hydroxynaphthalin-5-sulfonsäure, 2-Amino-5-hydroxynaphthalin-7-sulfonsäure, 2-Amino-6-hydroxynaphthalin-8-sulfonsäure, 2-Amino-8-hydroxynaphthalin-6-sulfonsäure, 2-Methylamino-5-hydroxynaphthalin-7-sulfonsäure, 2-Ethylamino-5-hydroxynaphthalin-7-sulfonsäure, 2-Methylamino-8-hydroxynaphthalin-6-sulfonsäure, 2-Ethylamino-8-hydroxynaphthalin-6-sulfonsäure, 1-Amino-6-hydroxynaphthalin-3,8-disulfonsäure, 1-Amino-8-hydroxynaphthalin-3,6-disulfonsäure, 1-Amino-8-hydroxynaphthalin-2,4-disulfonsäure, 1-Amino-8-hydroxynaphthalin4,6-disulfonsäure, 1-Amino-8-hydroxynaphthalin-3,5-disulfonsäure, 2-Amino-5-hydroxynaphthalin-1,7-disulfonsäure, 2-Amino-8-hydroxynaphthalin-3,6-disulfonsäure, 2-Amino-5-aminomethyl-naphthalin-1-sulfonsäure, 8-(4-Aminobenzoyl)amino-1-hydroxynaphthalin-3,5-disulfonsäure, 8-(2-Aminobenzoyl)amino-1-hydroxynaphthalin-3,5-disulfonsäure, 8-(4-Aminobenzoyl)amino-1-hydroxynaphthalin-3,6-disulfonsäure, 8-(2-Aminobenzoyl)amino-1-hydroxynaphthalin-3,6-disulfonsäure, 8-(3-Aminobenzoyl)amino-1-hydroxynaphthalin-3,6-disulfonsäure, 7-(4-Aminobenzoyl)amino-1-hydroxynaphthalin-3-sulfonsäure, 6-(2-Aminobenzoyl)amino-1-hydroxynaphthalin-3-sulfonsäure, 6-(4-Amino-2-sulfophenyl)amino-1-hydroxynaphthalin-3-sulfonsäure, 7-(4-Amino-2-sulfophenyl)amino-1-hydroxynaphthalin-3-sulfonsäure.

Geeignete chromophore Verbindungen des Typs (I) 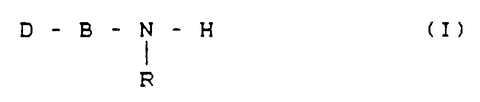 sind beispielsweise 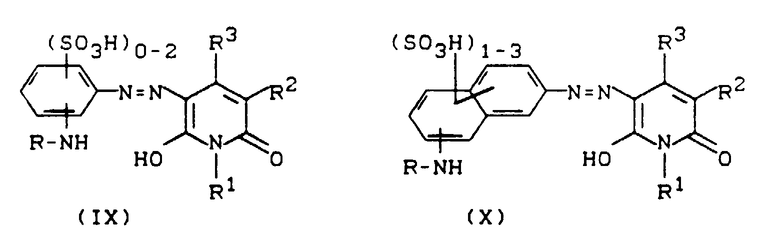 worin
- R: die oben angegebene Bedeutung hat,
- R¹ =: H, C₁-C₄-Alkyl, Aryl, β-Sulfoethyl,
- R² =: H, Cl, SO₃H, CONH₂, CH₂SO₃H, CH₃ und SO₂CH₃,
- R³ =: H, CH₃, CH₂-SO₃H, CO₂H, 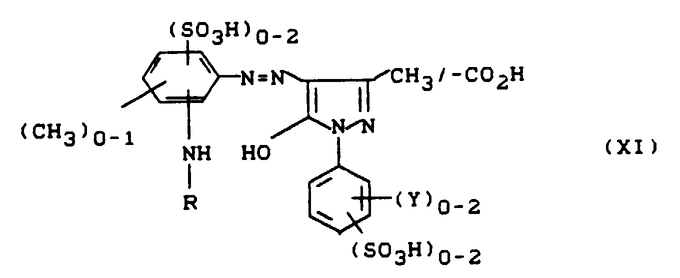
worin Y = Chlor oder Methyl, 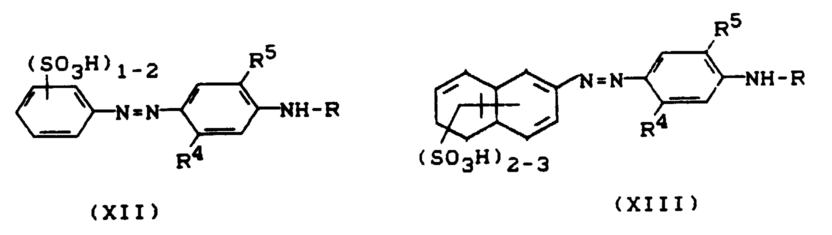 worin
- R⁴ =: H, Methyl, Ethyl, Methoxy, Ethoxy, Acetylamino, Ureido, gegebenenfalls subst. Phenylcarbonylamino, Mesylamino, Halogen,
- R⁵ =: H, Methyl, Ethyl, Methoxy, Ethoxy, Sulfo, 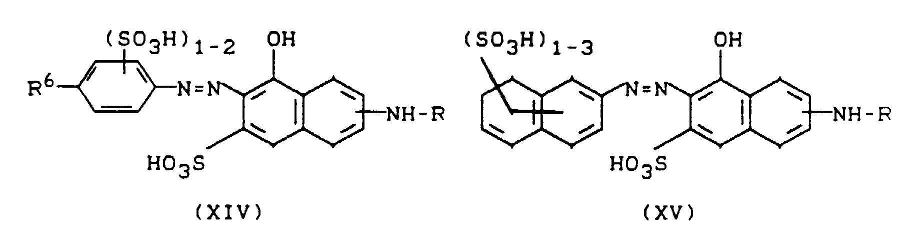 worin
- R⁶ =: H, Methyl, Ethyl, Methoxy, Ethoxy, Chlor oder Acetylamino, 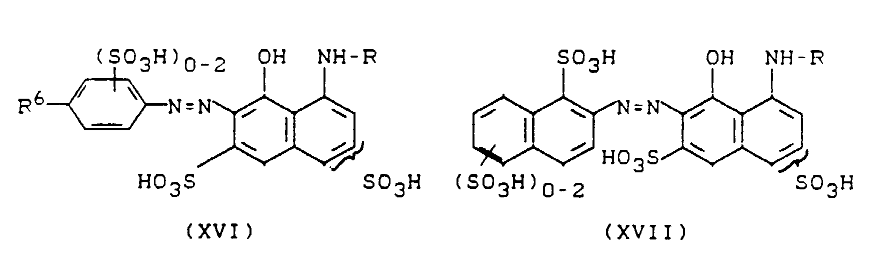
worin
- R⁶: die oben angegebene Bedeutung hat, 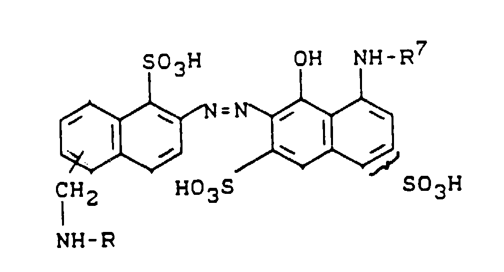 worin
- R⁷ =: 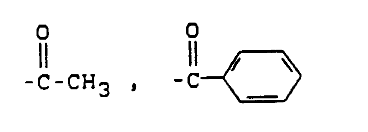 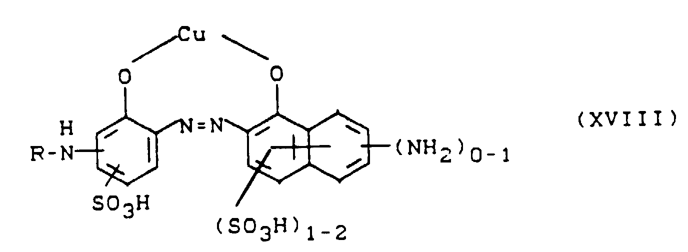 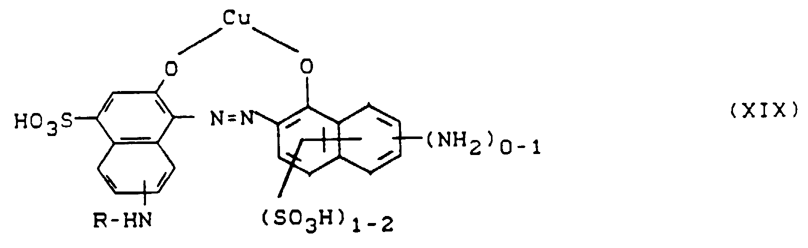 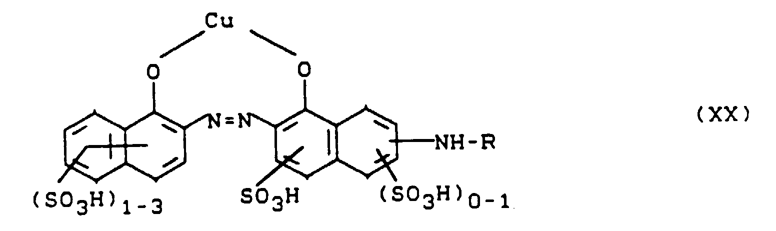 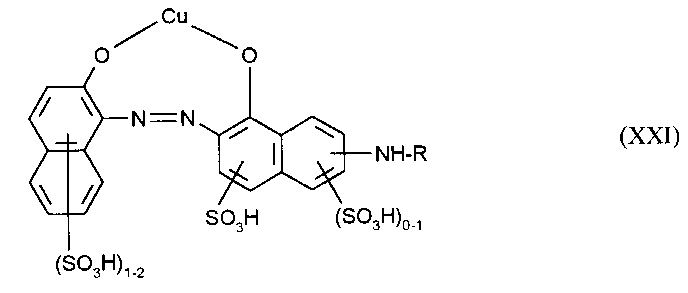 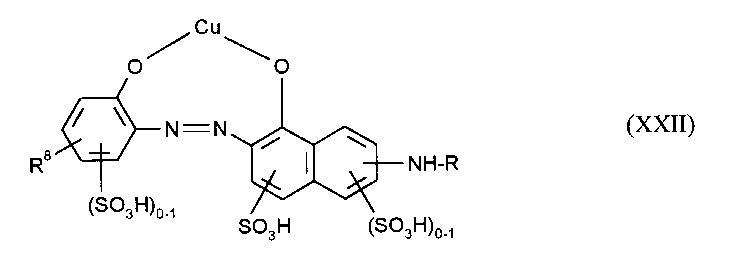 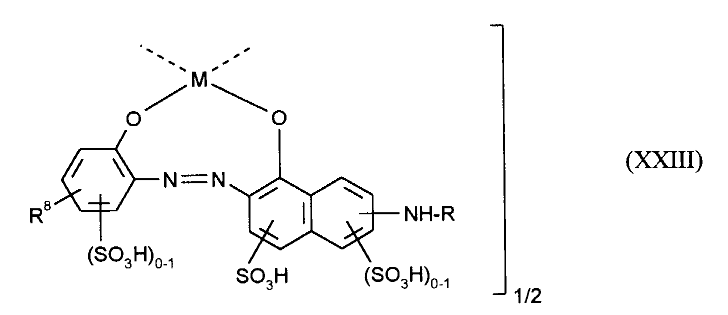
worin
- R⁸ =: H, Halogen, Nitro oder C₁-C₄-Alkyl und
- M =: Cr oder Co bedeutet und

a) 1:2 Cr- oder Co-Komplexe von (XXIII), die zwei gleiche Farbstoffe (XXIII) oder zwei verschiedene Farbstoffe (XXIII) enthalten, oder
b) 1:2 Cr- oder Co-Komplexe von (XXIII) mit einem beliebigen anderen metallkomplexbildenden Farbstoff, 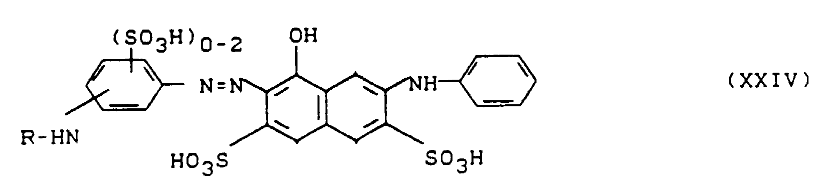 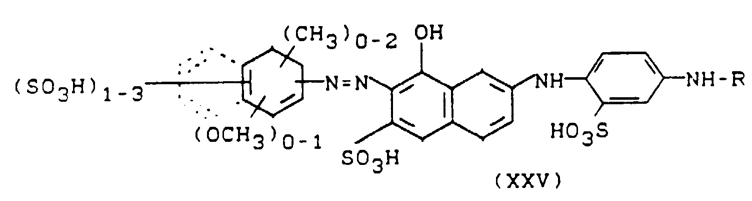 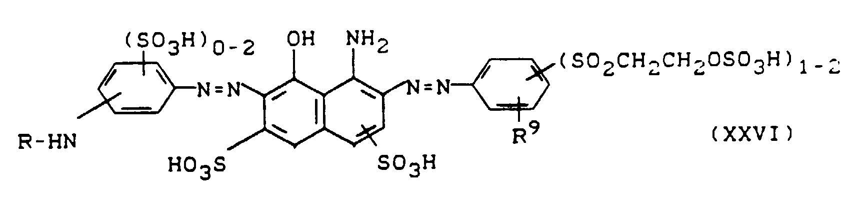
worin
- R⁹ =: H, Cl, Br, Methyl, Methoxy, Carboxy, Sulfo, 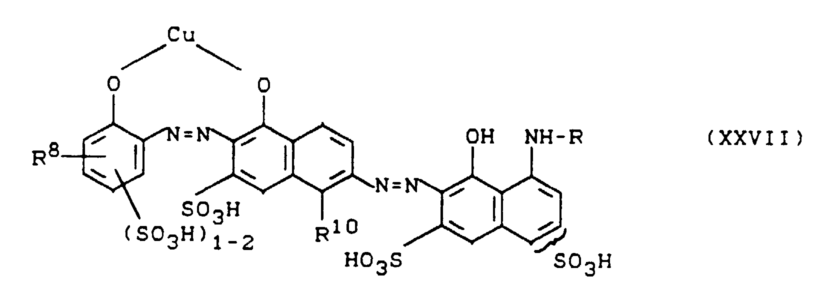
worin
- R¹⁰ =: H oder SO₃H und R⁸ die oben angegebene Bedeutung hat, 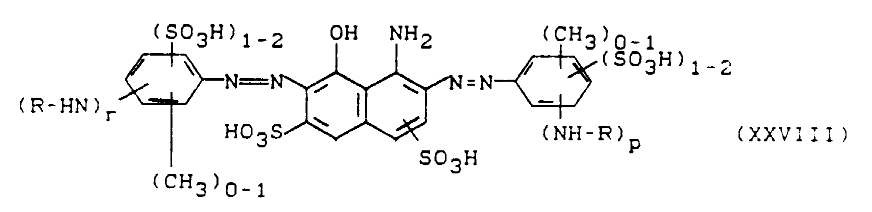
worin
r bzw. p = 0 oder 1, wobei die Summe
r + p = 1 oder 2, 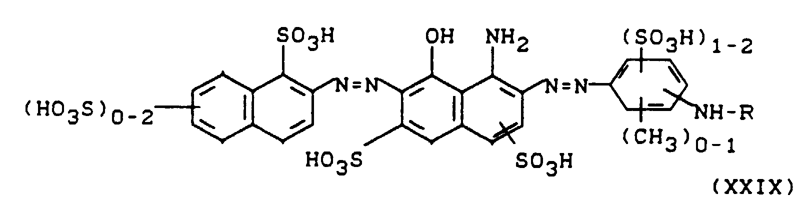 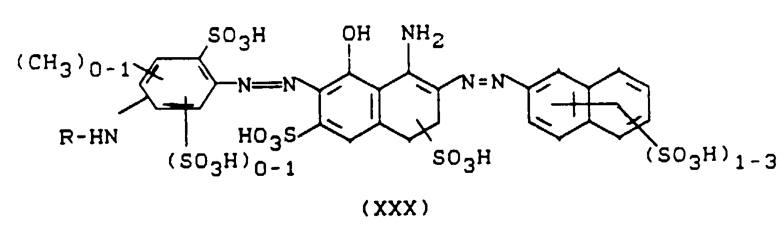 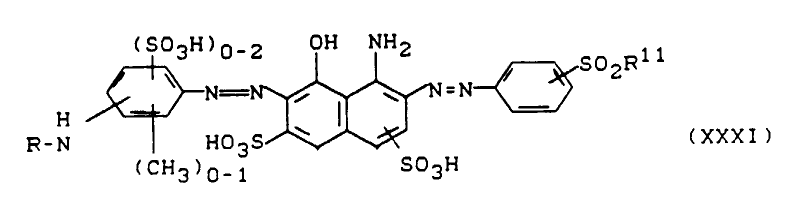 worin
- R¹¹ =: -NH₂, -NHCH₃, -CH₃, -C₂H₅, -CH₂CH₂OH, 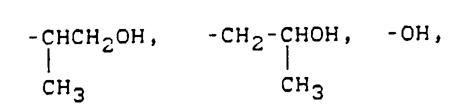 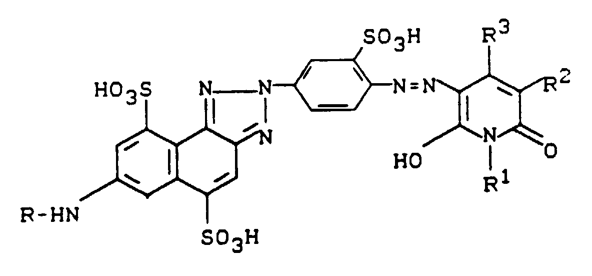
worin R¹, R², R³ die unter Formel (IX) und (X) angegebene Bedeutung haben, 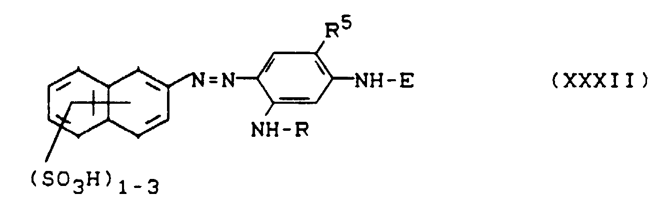 worin E = H, COCH₃ oder COC₆H₅ und R⁵ die unter Formel (XII) angegebene Bedeutung hat, 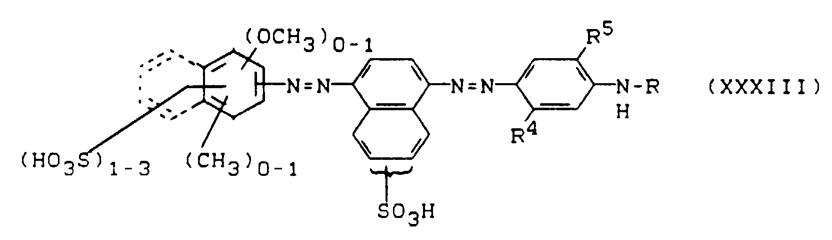 worin R⁴ und R⁵ die unter Formel (XII) angegebene Bedeutung hat, 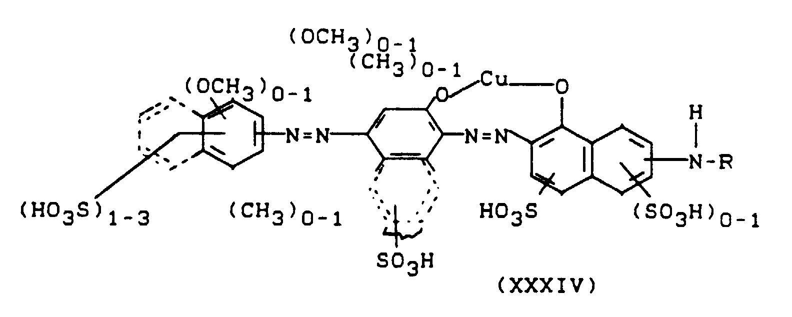 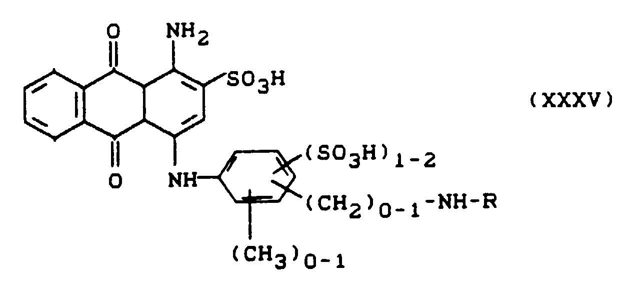 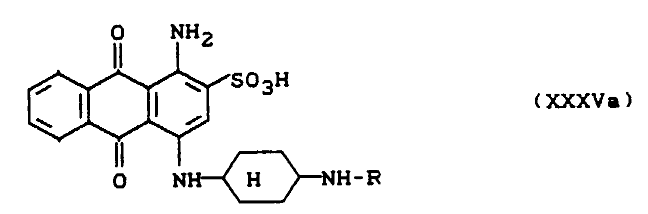 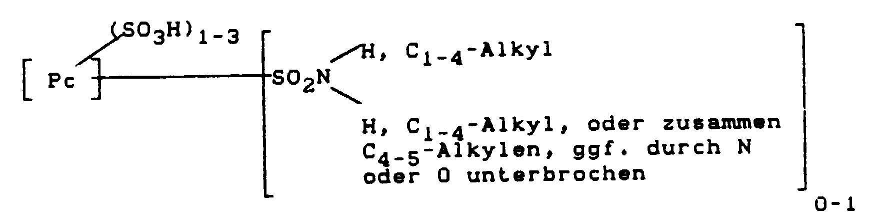 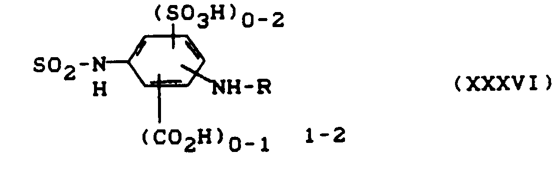 worin Pc für einen Cu- oder Ni-Phthalocyaninrest steht. Die Gesamtzahl der Substituenten am Pc-Gerüst ist dabei 4. 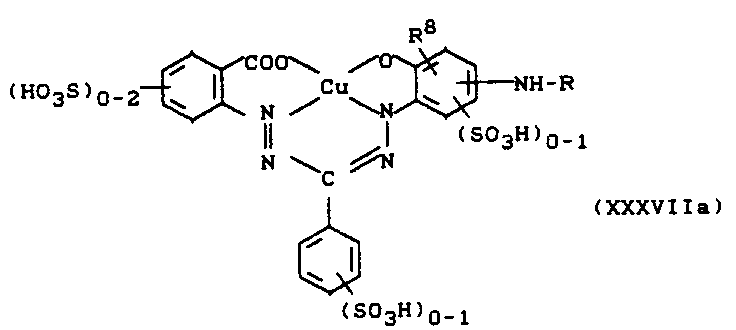 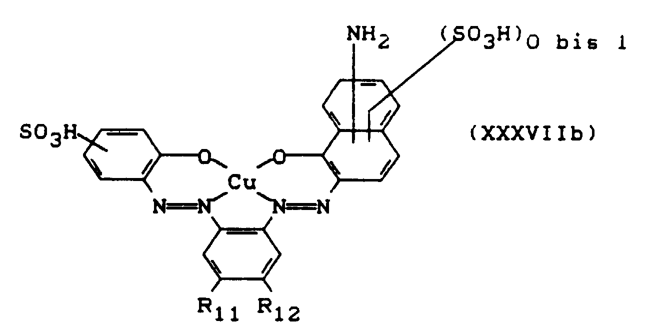 worin
- R₁₁ =: C₁-C₄-Alkyl, Halogen, -OC₁-C₄-Alkyl und
- R₁₂ =: OC₁-C₄-Alkyl 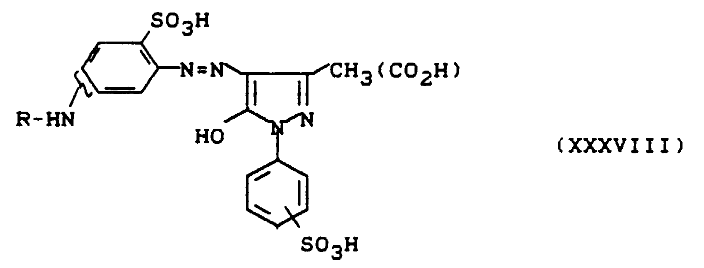 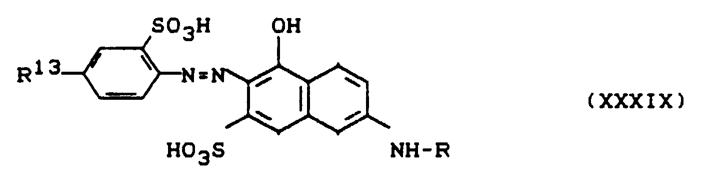
worin
- R¹³ =: H, CH₃, OCH₃, OC₂H₅ und NHCOCH₃ 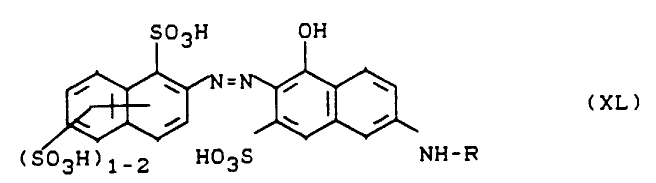 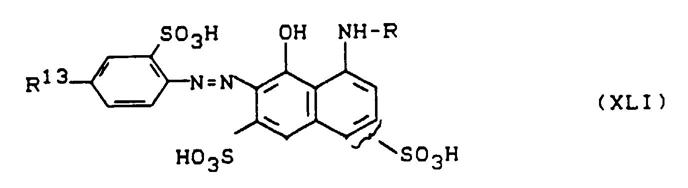
wobei R¹³ die unter Formel (XXXIX) genannte Bedeutung hat, 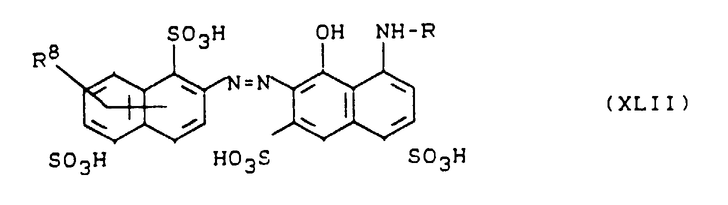 worin R⁸ die unter Formel (XXII) angegebene Bedeutung hat,

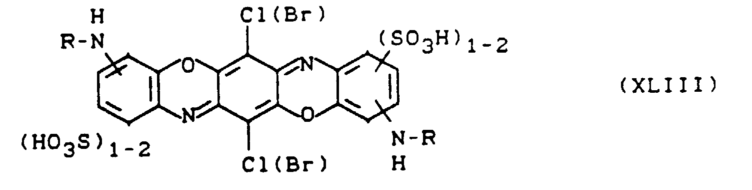

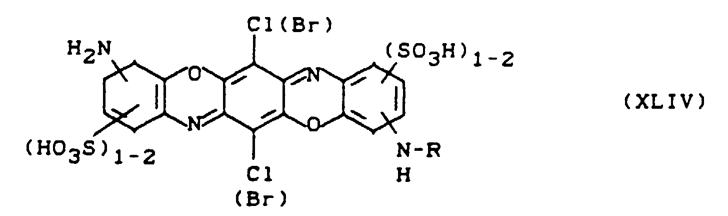

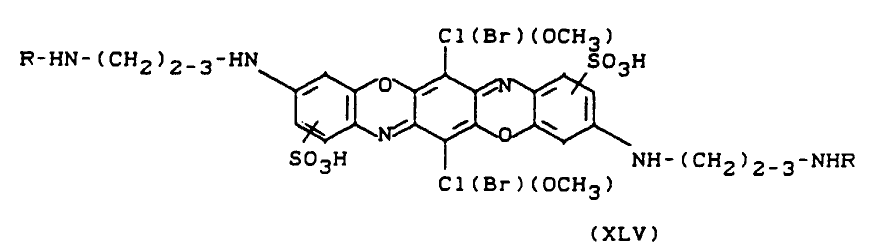

Geeignete Halogenpyrimidinreste Z sind:
2,4-Dichlorpyrimidinyl-6-, 2,4,5-Trichlorpyrimidinyl-6-, 2,4-Dichlor-5-nitro- oder -5-methyl- oder -5-carboxymethyl- oder -5-carboxy- oder -5-cyano- oder -5-vinyl- oder -5-sulfo- oder -5-mono-, -di- oder -trichlormethyl- oder -5-carbalkoxy-pyrimidinyl-6- sowie die entsprechenden Brom- und Fluor-Derivate der oben erwähnten Pyrimidinylresten, unter diesen beispielsweise 2-Fluor-4-pyrimidinyl-, 5,6-Difluor-4-pyrimidinyl-, 6-Fluor-5-chlor-4-pyrimidinyl-, 2,6-Difluor-4-pyrimidinyl-, 2,6-Difluor-5-chlor-4-pyrimidinyl-, 2-Fluor-5,6-dichlor-4-pyrimidinyl-, 2,6-Difluor-5-methyl-4-pyrimidinyl-, 2-Fluor-5-chlor-6-methyl-4-pyrimidinyl-, 2-Fluor-5-methyl-6-chlor-4-pyrimidinyl-, 2-Fluor-5-nitro-6-chlor-4-pyrimidinyl-, 5-Brom-2-fluor-4-pyrimidinyl-, 2-Fluor-5-cyan-4-pyrimidinyl-, 2-Fluor-5-methyl-4-pyrimidinyl-, 2,5,6-Trifluor-4-pyrimidinyl-, 5-Chlor-6-chlormethyl-2-fluor-4-pyrimidinyl-, 5-Chlor-6-dichlormethyl-2-fluor-4-pyrimidinyl-, 5-Chlor-6-trichlormethyl-2-fluor-4-pyrimidinyl-, 5-Chlor-2-chlormethyl-6-fluor-4-pyrimidinyl-, 5-Chlor-2-dichlormethyl-6-fluor-4-pyrimidinyl-, 5-Chlor-2-trichlormethyl-6-fluor-4-pyrimidinyl-, 5-Chlor-2-fluordichlormethyl-6-fluor-4-pyrimidinyl-, 2,6-Difluor-5-brom-4-pyrimidinyl-, 2-Fluor-5-brom-6-methyl-4-pyrimidinyl-, 2-Fluor-5-brom-6-chlormethyl-4-pyrimidinyl-, 2,6-Difluor-5-chlormethyl-4-pyrimidinyl-, 2,6-Difluor-5-nitro-4-pyrimidinyl-, 2-Fluor-6-methyl-4-pyrimidinyl-, 2-Fluor-5-chlor-6-methyl-4-pyrimidinyl-, 2-Fluor-5-chlor-4-pyrimidinyl-, 2-Fluor-6-chlor-4-pyrimidinyl-, 6-Trifluormethyl-5-chlor-2-fluor-4-pyrimidinyl-, 6-Trifluormethyl-2-fluor-4-pyrimidinyl-, -fluor-4-pyrimidinyl-, 2-Fluor-5-trifluormethyl-4-pyrimidinyl-, 2-Fluor-5-phenyl- oder -5-methylsulfonyl-4-pyrimidinyl-, 2-Fluor-5-carbonamido-4-pyrimidinyl-, 2-Fluor-5-carbmethoxy-4-pyrimidinyl-, 2-Fluor-5-brom-6-trifluormethyl-4-pyrimidinyl-, 2-Fluor-6-carbonamido-4-pyrimidinyl-, 2-Fluor-6-carbmethoxy-4-pyrimidinyl-, 2-Fluor-6-phenyl-4-pyrimidinyl-, fluormethyl-4-pyrimidinyl-, 5-Chlor-6-Fluor-2-Methyl-4-pyrimidinyl-, 5,6-Difluor-2-Trifluormethyl-4-pyrimidinyl-, 5-Chlor-6-Fluor-2-Dichlorfluormethyl-4-pyrimidinyl-, 2-Fluor-5-chlorpyrimidin-4-yl, 2-Methyl-4-fluor-5-methylsulfonylpyrimidinyl-6, 2,6-Difluor-5-methyl-sulfonyl-4-pyrimidinyl-, 2,6-Dichlor-5-methylsulfonyl-4-pyrimidinyl, 2-Fluor-5-sulfonamido-4-pyrimidinyl-, 2-Fluor-5-chlor-6-carbomethoxy-4-pyrimidinyl-, 2,6-Difluor-5-trifluormethyl-4-pyrimidinyl; sulfo nylgruppenhaltige Triazinreste, wie 2,4-Bis-(phenylsulfonyl)-triazinyl-6-, 2-(3'-Carboxyphenyl)-sulfonyl-4-chlortriazinyl-6-, 2-(3'-Sulfophenyl)-sulfonyl-4-chlortriazinyl-6-, 2,4-Bis-(3'-carboxyphenylsulfonyl)-triazinyl-6; sulfonylgruppenhaltige Pyrimidinringe, wie 2-Carboxymethylsulfonyl-pyrimidinyl-4-, 2-Methylsulfonyl-6-methyl-pyrimidinyl-4-, 2-Methylsulfonyl-6-ethyl-pyrimidinyl-4-, 2-Phenylsulfonyl-5-chlor-6-methyl-pyrimidinyl-4-, 2,6-Bis-methylsulfonyl-pyrimidinyl-4-, 2,6-Bis-methylsulfonyl-5-chlor-pyrimidinyl-4-, 2,4-Bis-methylsulfonyl-pyrimidin-5-sulfonyl-, 2-Methylsulfonyl-pyrimidinyl-4-, 2-Phenylsulfonyl-pyrimidinyl-4-, 2-Trichlormethylsulfonyl-6-methyl-pyrimidinyl-4-, 2-Methylsulfonyl-5-chlor-6-methyl-pyrimidinyl-4-, 2-Methylsulfonyl-5-brom-6-methyl-pyrimidinyl-4-, 2-Methylsulfonyl-5-chlor-6-ethyl-pyrimidinyl-4-, 2-Methylsulfonyl-5-chlor-6-chlormethyl-pyrimidinyl-4-, 2-Methylsulfonyl-4-chlor-6-methylpyrimidin-5-sulfonyl-, 2-Methylsulfonyl-5-nitro-6-methylpyrimidinyl-4-, 2,5,6-Tris-methylsulfonyl-pyrimidinyl-4-, Methylsulfonyl-6-chlor-pyrimidinyl-4-, 2-Ethylsulfonyl-5-chlor-6-methyl-pyrimidinyl-4-, sulfonyl-5-chlor-pyrimidinyl-4-, 2,6-Bis-methylsulfonyl-5-chlor-pyrimidinyl-4-, 2-Methylsulfonyl-6-carboxypyrimidinyl-4-, 2-Methylsulfonyl-5-sulfo-pyrimidinyl-4-, 2-Methylsulfonyl-6-carbomethoxy-pyrimidinyl-4-, 2-Methylsulfonyl-5-carboxy-pyrimidinyl-4-, 2-Methylsulfonyl-5-cyan-6-methoxy-pyrimidinyl-4, sulfonyl-6-methyl-pyrimidinyl-4-, 2-β-Sulfoethylsulfonyl-6-methyl-pyrimidinyl-4-, 2-Methylsulfonyl-5-brom-pyrimidinyl-4-, 2-Phenylsulfonyl-5-chlor-pyrimidinyl-4-, 2-Carboxymethylsulfonyl-5-chlor-6-methyl-pyrimidinyl-4-, 2-Methylsulfonyl-6-chlorpyrimidin-4.

Besonders geeignet sind die Halogenpyrimidine (Hal-Z) der Formeln:

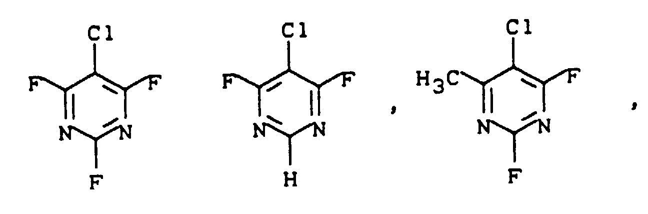

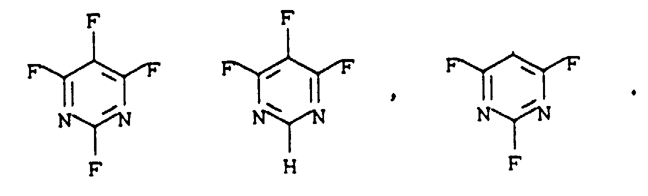

### Beispiel 1

In einem Strahldüsenreaktor nach Bild 1 werden gleichzeitig und kontinuierlich über getrennte Zuleitungen 9 kg/Std. 5-Chlor-2,4,6-trifluorpyrimidin von 20°C sowie 171 l/Std. einer wäßrigen 40°C warmen Lösung von 12,9 kg 7-Amino-4-hydroxynaphthalin-2-sulfonsäure Na-Salz und 2,1 kg Natriumfluorid so eingespeist, daß das 5-Chlor-2,4,6-trifluorpyrimidin mit einem Druckabfall von 35 bar in die strömende wäßrige Lösung eintritt. Nach Austritt aus dem Strahldüsenreaktor wird die Reaktion in einer Verweilzeitstrecke oder in einer Rührwerkskesselkaskade zu Ende geführt. Die so erhaltene Lösung wird nach Abkühlen auf 0°C auf dem üblichen Weg mit dem Diazoniumsalz aus 2-Amino-5-methoxy-benzolsulfonsäure umgesetzt und liefert nach dem Aussalzen mit Natriumchlorid in guten Ausbeuten den Reaktivfarbstoff der Formel 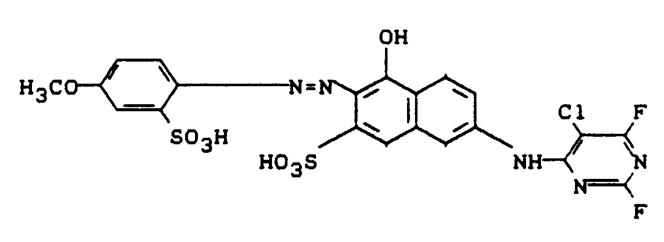 der Baumwolle in klaren scharlachfarbenen Tönen färbt.

### Beispiel 2

In einer Dispergiereinheit des Typs Rotor/Stator-Mischer werden gleichzeitig und kontinuierlich 9 kg/Std. 5-Chlor-2,4,6-trifluorpyrimidin von 20°C sowie 120 l/Std einer wäßrigen 30°C warmen Lösung von 12,48 kg 2-Amino-5-aminomethyl-naphthalin-1-sulfonsäure und 4,4 kg Natriumhydroxid eingespeist. Nach dem Austritt aus dem Rotor/Stator-Mischer wird die Reaktion in einer Verweilzeitstrecke bzw. in einem Rührwerksbehälter zu Ende geführt. Nach Abkühlen auf 0°C wird die so erhaltene Suspension mit Salzsäure auf pH ca. 1,5 gestellt und mit Natriumnitrit versetzt. Das so erhaltene Diazoniumsalz wird auf üblichen Weg auf 8-Benzoylamino-1-hydroxynaphthalin-3,6-disulfonsäure gekuppelt. Nach der Isolierung erhält man in guten Ausbeuten den Reaktivfarbstoff der Formel 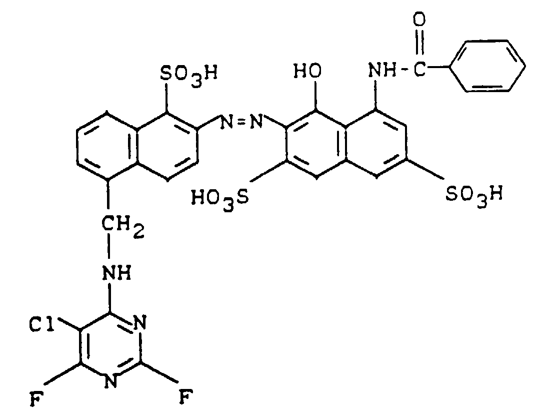 der Baumwolle in roten Tönen mit guten Echtheiten färbt.

### Beispiel 3

In einem Strahldüsenreaktor nach Bild 1 werden gleichzeitig und kontinuierlich über getrennte Zuläufe 9,5 kg/Std. 4,6-Difluor-5-chlorpyrimidin mit einer Temperatur von 20°C und 188 l/Std. einer wäßrigen Lösung von 50°C, enthaltend 11,3 kg 1,3-Diamino-benzol-4-sulfonsäure und 2,4 kg Natriumhydroxid so eingeleitet, daß das 4,6-Difluor-5-chlorpyrimidin mit einem Druckabfall von 30 bar in die strömende Lösung eintritt. Nach dem Austritt aus dem Reaktor wird die Reaktion in einer Verweilzeitstrecke oder in einer Kesselkaskade zu Ende geführt. Danach wird auf üblichem Weg mit Kochsalz ausgesalzen und isoliert oder ohne Isolierung weitergearbeitet.

Das Zwischenprodukt mit der folgenden Formel 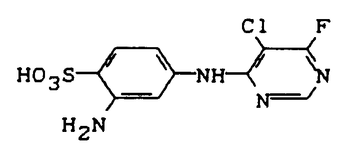 wird nach üblichem Verfahren für Reaktivfarbstoffe eingesetzt.

### Beispiel 4

In einem Strahldüsenreaktor nach Bild 1 werden gleichzeitig und kontinuierlich über getrennte Zuläufe 9,0 kg/Std. 2,4,6-Trifluorpyrimidin mit einer Temperatur von 20°C und 430 l/Std. einer wäßrigen Lösung von 50°C, enthaltend 41,7 kg Farbbase der Formel 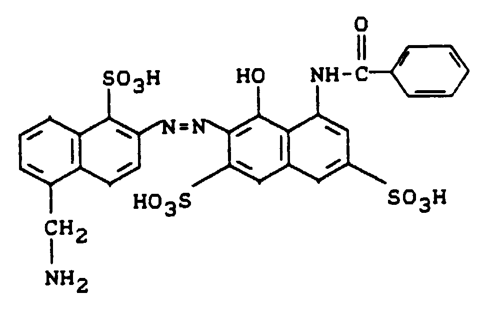 und 9,6 kg Natriumhydroxid so eingeleitet, daß das 2,4,6-Trifluorpyrimidin mit einem Druckabfall von 30 bar in die strömende Lösung eintritt. Nach dem Austritt aus dem Reaktor wird die Reaktion in einer Verweilzeitstrecke oder in einer Kesselkaskade zu Ende geführt. Danach wird auf üblichem Weg mit Kochsalz ausgesalzen und isoliert.

Der Farbstoff der Formel 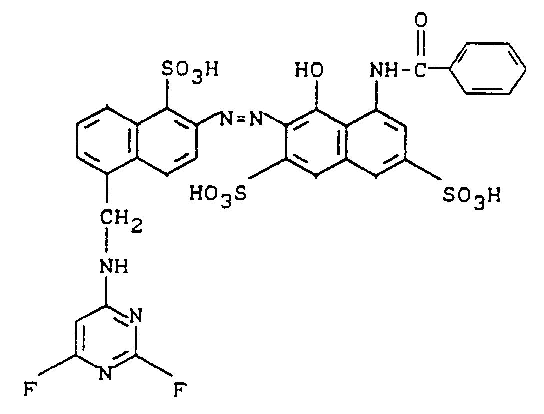 färbt Cellulose in roten Tönen mit sehr hoher Naßechtheit.

### Beispiel 5

In einem Strahldüsenreaktor nach Bild 1 werden gleichzeitig und kontinuierlich über getrennte Zuläufe 9,5 kg/Std. 5-Chlor-2,4,6-Trifluorpyrimidin mit einer Temperatur von 20°C und 455 l/Std. einer wäßrigen Lösung von 45°C, enthaltend 35,9 kg Dinatriumsalz der Farbbase der Formel 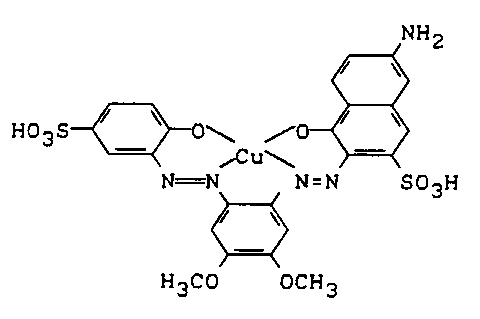 und 7,2 kg Dinatriumhydrogenphosphat so eingeleitet, daß das 5-Chlor-2,4,6-Trifluorpyrimidin mit einem Druckabfall von 30 bar in die strömende Lösung eintritt. Nach dem Austritt aus dem Reaktor wird die Reaktion in einer Verweilzeitstrecke oder in einer Kesselkaskade zu Ende geführt. Danach wird auf üblichem Wege mit Kochsalz ausgesalzen und isoliert.

Der Farbstoff der Formel 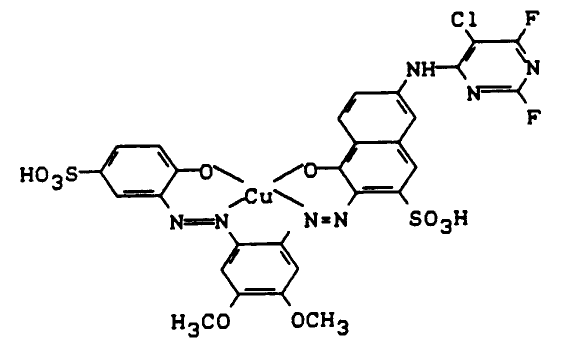 färbt Cellulose in oliven Tönen mit sehr hoher Naßechtheit.

### Beispiel 6

In einem Strahldüsenreaktor nach Bild 1 werden gleichzeitig und kontinuierlich über getrennte Zuläufe 9,5 kg/Std. 5-Chlor-2,4,6-trifluorpyrimidin mit einer Temperatur von 20°C und 118 l/Std. einer wäßrigen Lösung von 40°C, enthaltend 29,3 kg Dinatriumsalz der Farbbase der Formel 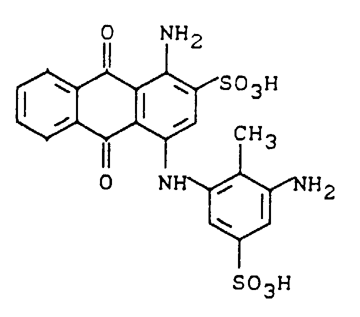 und 2,2 kg Natriumhydroxid so eingeleitet, daß das 5-Chlor-2,4,6-trifluorpyrimidin mit einem Druckabfall von 30 bar in die strömende Lösung eintritt. Nach dem Austritt aus dem Reaktor wird die Reaktion in einer Verweilzeitstrecke oder in einer Kesselkaskade zu Ende geführt. Danach wird auf üblichem Weg mit Kochsalz ausgesalzen und isoliert.

Der Farbstoff der Formel 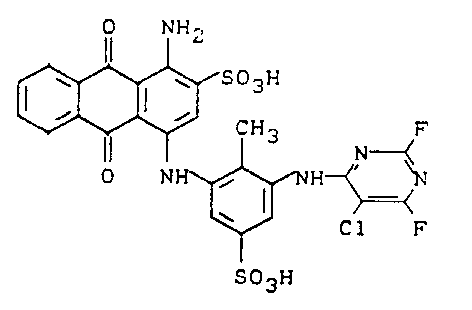 färbt Cellulose in blauen Tönen mit sehr hoher Naßechtheit.

### Beispiel 7

In einem Strahldüsenreaktor nach Bild 1 werden gleichzeitig und kontinuierlich über getrennte Zuläufe 9,5 kg/Std. 5-Chlor-2,4,6-trifluorpyrimidin mit einer Temperatur von 20°C und 50 l/Std. einer wäßrigen Lösung von 30°C, enthaltend 5,79 kg 1,3-Diamino-benzol und pH 7,0 so eingeleitet, daß das 5-Chlor-2,4,6-trifluorpyrimidin mit einem Druckabfall von 30 bar in die strömende Lösung eintritt. Nach dem Austritt aus dem Reaktor wird die Reaktion in einer Verweilzeitstrecke oder in einer Kesselkaskade zu Ende geführt. Danach wird auf üblichem Weg mit der Farbbase folgender Struktur 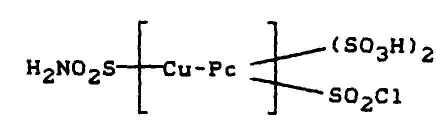 umsetzt zum Reaktivfarbstoff der Formel 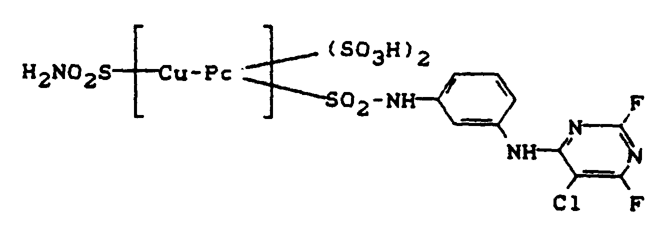 der Cellulose in türkisblauen Tönen mit sehr hoher Naßechtheit färbt.

## Patentansprüche

1. Verfahren zur kontinuierlichen Umsetzung von Halogenpyrimidinen mit Aminen, wobei Halogenpyrimidin und eine wäßrige Aminlösung bzw. Suspension über getrennte Zuleitungen in einen Reaktor eingeleitet werden und das Reaktionsprodukt anschließend abgeleitet wird, dadurch gekennzeichnet, daß die Edukte gleichzeitig und kontinuierlich unter intensiver Vermischung in den Reaktor eingeleitet werden.

2. Verfahren gemäß Anspruch 1, dadurch gekennzeichnet, daß man als Reaktor eine Dispergiereinheit vom Typ Rotor-Stator-Mischer verwendet, wobei das Halogenpyrimidin mit einer Reynolds-Zahl von mindestens 10.000 und die wäßrige Aminlösung mit einer solchen von mindestens 2.500 in den Reaktor einströmen.

3. Verfahren gemäß Anspruch 1, dadurch gekennzeichnet, daß man als Reaktor ein Reaktionsrohr verwendet, in welches eine oder mehrere Düsen einmünden, wobei die Aminlösung über das Reaktionsrohr mit einer Reynolds-Zahl von mindestens 2.500, das Halogenpyrimidin über die Düse bzw. Düsen mit einer Reynolds-Zahl von mindestens 10.000, in den Reaktor einströmen, wobei die Differenz der Strömungsgeschwindigkeiten zwischen Halogenpyrimidinstrom und Aminlösungsstrom mindestens 20 m/s, vorzugsweise 40 m/s beträgt.

4. Verfahren nach Anspruch 3, dadurch gekennzeichnet, daß ein Verhältnis des Reaktorquerschnittes F_{R} zum Einströmquerschnitt F_{I} des Halogenpyrimidinstroms von 225 bis 40.000, vorzugsweise von 700 bis 12.000, eingehalten wird.

5. Verfahren nach wenigstens einem der vorhergehenden Ansprüche, dadurch gekennzeichnet, daß ein Halogenpyrimidin Hal-Z mit einem Amin der Formel 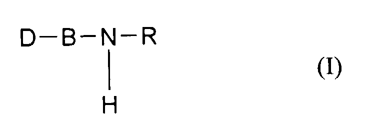 zu Reaktivfarbstoffen bzw. Reaktivfarbstoff-Vorprodukten der Formel 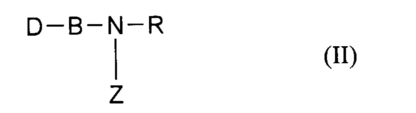 umgesetzt wird,
worin
B für direkte Bindung oder Brückenglied an ein aromatisch-carbocyclisches C-Atom des Restes D,
R für H, gegebenenfalls substituiertes C₁-C₄-Alkyl gegebenenfalls substituiertes Phenyl,
D für einen Rest der Benzol-, Naphthalin- oder heterocyclischen Reihe, insbesondere für sulfonsäuregruppenhaltige Reste von aromatischen Aminen, die als Diazo- bzw. Kupplungskomponenten verwendet werden oder für den Rest eines Chromophors, insbesondere für den Rest eines Farbstoffs der Mono- oder Polyazo-, Metallkomplexazo-, Anthrachinon-, Phthalocyanin-, Formazan, Azomethin-, Dioxazin-, Phenazin-, Xanthen-, Thioxanthon, Naphthochinon-, Stilben- oder Triphenylmethan-Reihe steht,
Z für Mono-, Di- und Trihalogenpyrimidinylreste,
Hal für -F, -Cl und -Br steht.

6. Verfahren nach wenigstens einem der vorhergehenden Ansprüche, dadurch gekennzeichnet, daß das Amin wenigstens einen der folgenden Reste aufweist

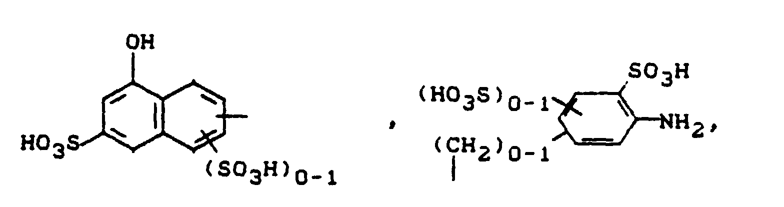

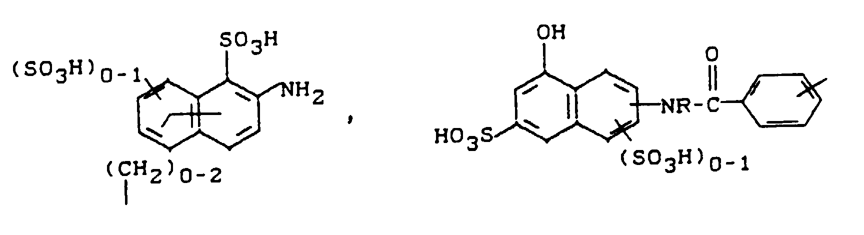

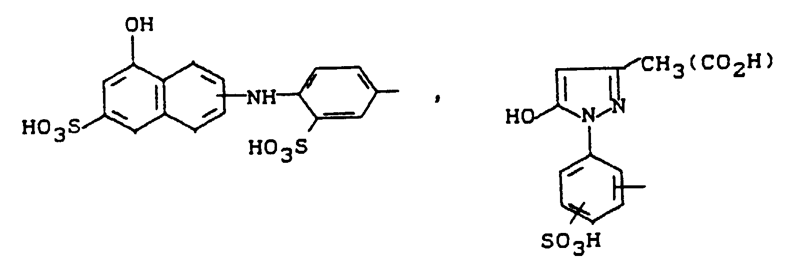

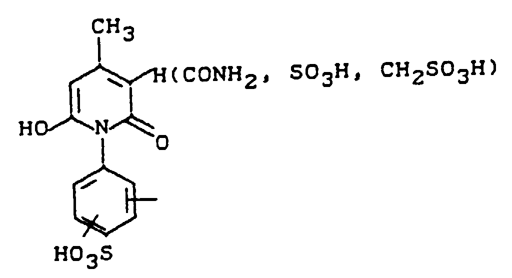

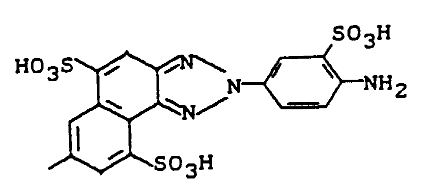

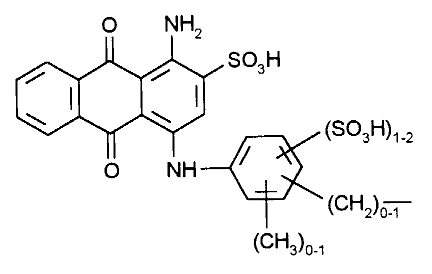

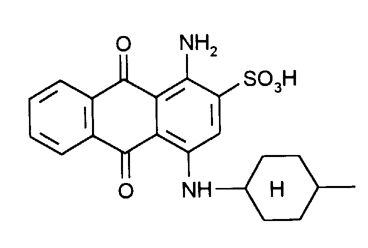

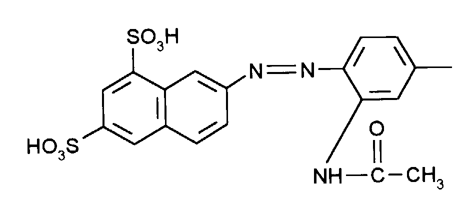

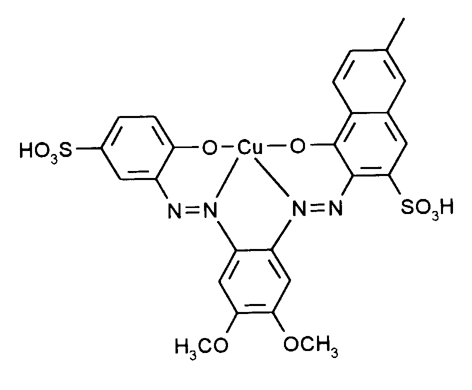

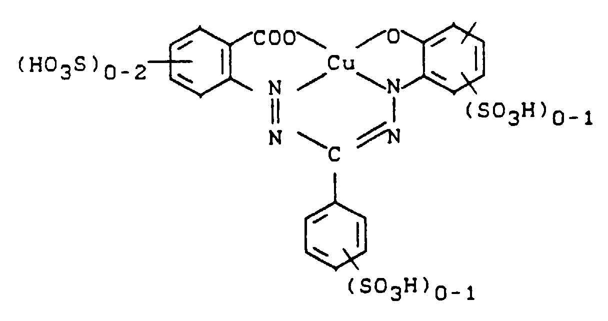

7. Verfahren nach wenigstens einem der vorhergehenden Ansprüche, dadurch gekennzeichnet, daß das Halogenpyrimidin wenigstens einer der folgenden Formeln entspricht

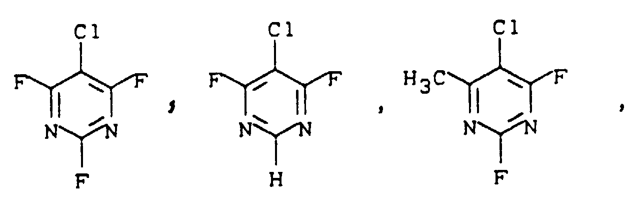

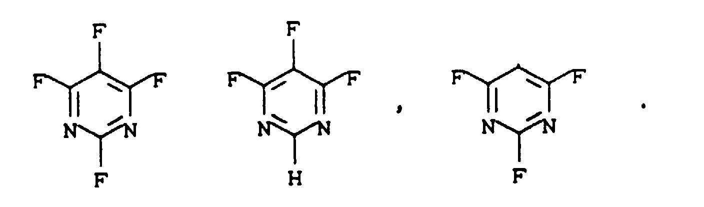

## Claims

1. Process for the continuous reaction of halogenopyrimidine with amines, halogenopyrimidine and an aqueous amine solution or suspension being passed into a reactor via separate feed lines and the reaction product being subsequently conducted away, characterised in that the starting materials are introduced into the reactor simultaneously and continuously with intensive mixing.

2. Process according to Claim 1, characterised in that the reactor used is a dispersion unit of the rotor/ stator mixer type, the halogenopyrimidine flowing into the reactor with a Reynolds number of at least 10,000 and the aqueous amine solution flowing into the reactor with a Reynolds number of at least 2,500.

3. Process according to Claim 1, characterised in that the reactor used is a reaction tube into which open one or more nozzles, the amine solution flowing into the reactor via the reaction tube with a Reynolds number of at least 2,500, the halogenopyrimidine flowing into the reactor via the nozzle or nozzles with a Reynolds number of at least 10,000, the difference in flow velocities between the halogenopyrimidine stream and amine solution stream being at least 20 m/s, preferably 40 m/s.

4. Process according to Claim 3, characterised in that a ratio of reactor cross-sectional area F_{R} to inlet cross-sectional area F₁ of the halogenopyrimidine stream of 225 to 40,000, preferably 700 to 12,000, is maintained.

5. Process according to at least one of the preceding claims, characterised in that a halogenopyrimidine Hal-Z is reacted with an amine of the formula 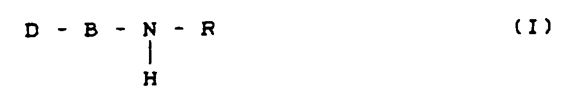 to give reactive dyes or reactive dye precursors of the formula 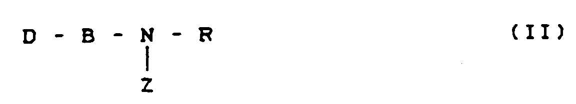 in which
B represents a direct linkage or bridge member to an aromatic carbocyclic C atom of the radical D,
R represents H, unsubstituted or substituted C₁-C₄-alkyl, unsubstituted or substituted phenyl,
D represents a radical of the benzene, naphthalene or heterocyclic series, in particular sulpho group-containing radicals of aromatic amines, which are used as diazo components or coupling components, or the radical of a chromophore, in particular the radical of a dye of the monoazo or polyazo, metal complex azo, anthraquinone, phthalocyanine, formazan, azomethine, dioxazine, phenazine, xanthene, thioxanthone, naphthoquinone, stilbene or triphenylmethane series,
Z represents mono-, di- and trihalogenopyrimidinyl radicals,
Hal represents -F, -Cl and -Br.

6. Process according to at least one of the preceding claims, characterised in that the amine has at least one of the radicals below

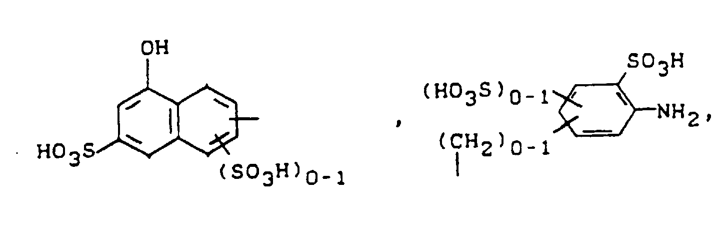

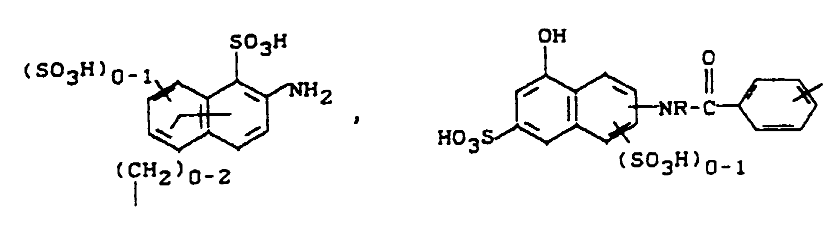

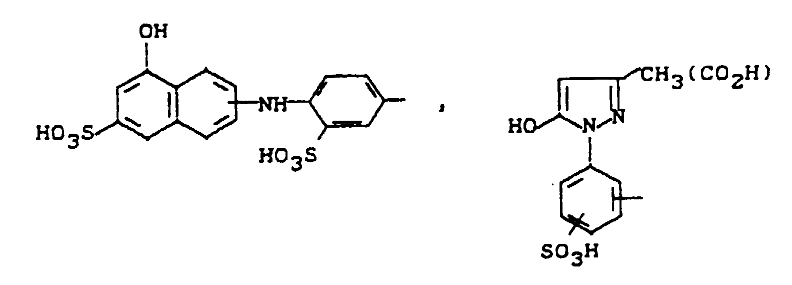

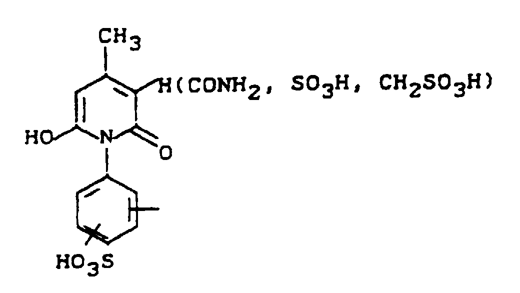

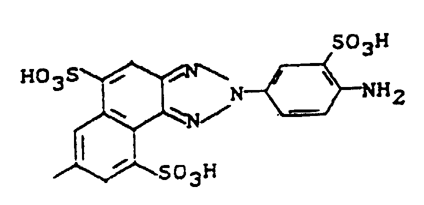

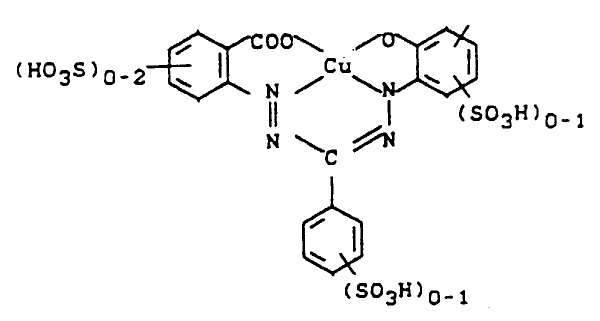

7. Process according to at least one of the preceding claims, characterised in that the halogenopyrimidine corresponds to at least one of the formulae below

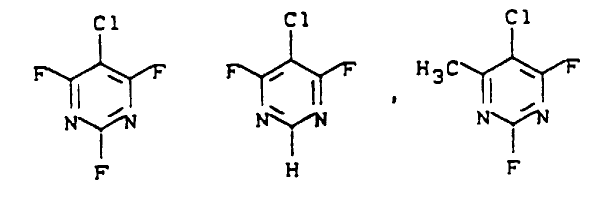

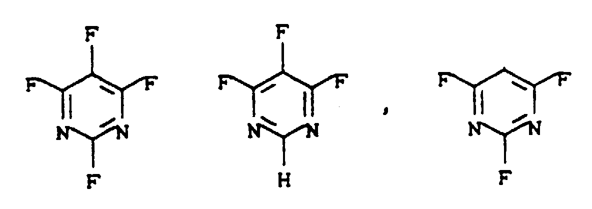

## Revendications

1. Procédé de réaction en continu d'halogénopyrimidines avec des amines, où on introduit l'halogénopyrimidine et une solution ou une suspension aqueuse d'amine par des conduites séparées dans un réacteur et on soutire le produit de réaction ensuite, caractérisé en ce qu'on introduit simultanément et en continu les éduits sous mélange énergique dans le réacteur.

2. Procédé selon la revendication 1, caractérisé en ce qu'on utilise comme réacteur une unité de dispersion du type mélangeur rotor-stator, où on introduit l'halogénopyrimidine avec nombre de Reynolds d'au moins 10 000 et la solution aqueuse d'amine avec un nombre de Reynolds d'au moins 2 500 dans le réacteur.

3. Procédé selon la revendication 1, caractérisé en ce qu'on utilise comme réacteur un réacteur tubulaire, où une ou plusieurs buses débouchent, où la solution d'amine s'écoule dans le réacteur tubulaire avec un nombre de Reynolds d'au moins 2 500, l'halogénopyrimidine s'écoule par la buse ou les buses avec un nombre de Reynolds d'au moins 10 000, où la différence des vitesses d'écoulement entre le courant d'halogénopyrimidine et le courant de la solution d'amine présente au moins 20 m/s, de préférence 40 m/s.

4. Procédé selon la revendication 3, caractérisé en ce qu'on établit un rapport de la section du réacteur F_{R} à la section d'injection R_{I} du courant d'halogénopyrimidine de 225 à 40 000, de préférence 700 à 12 000.

5. Procédé selon au moins l'une des revendications précédentes, caractérisé en ce que qu'on fait réagir une halogénopyrimidine Hal-Z avec une amine de formule 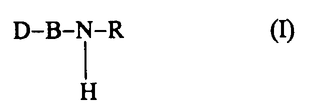 pour donner des colorants réactifs ou des précurseurs de colorants réactifs de formule 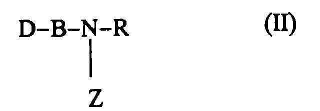 où
B représente la fixation directe ou est un organe de pont sur l'atome de carbone carbocyclique aromatique du reste D,
R représente H, un phényle le cas échéant substitué par un alkyle C₁-C₄ substitué le cas échéant,
D représente un reste de la série hétérocyclique, de benzène ou de naphtalène, notamment des restes contenant des groupes acides sulfoniques d'amines aromatiques, qu'on utilise comme composants de diazotation ou de couplage ou le reste d'un chromophore, notamment le reste d'un colorant de la série des mono- ou polyazoïques, des azoïques complexes métallifères, d'anthraquinone, de phtalocyanine, de formazane, d'azométhine, de dioxazine, de phénazine, de xanthène, de thioxanthone, de naphtoquinone, de stilbène ou de triphénylméthane,
Z représente des restes mono-, di- et trihalogénopyrimidinyle,
Hal représente -F, -Cl et -Br.

6. Procédé selon au moins l'une des revendications précédentes, caractérisé en ce que l'amine présente au moins l'un des restes suivants :

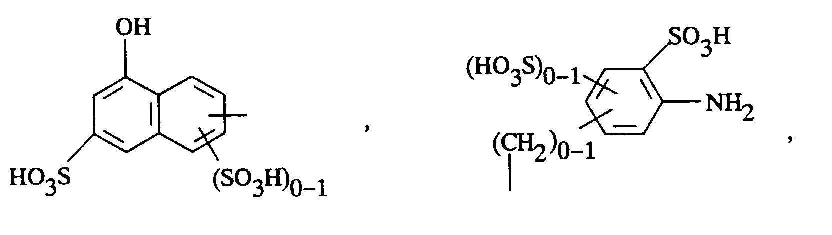

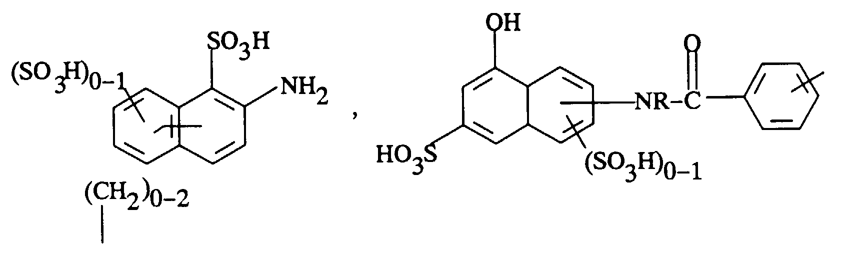

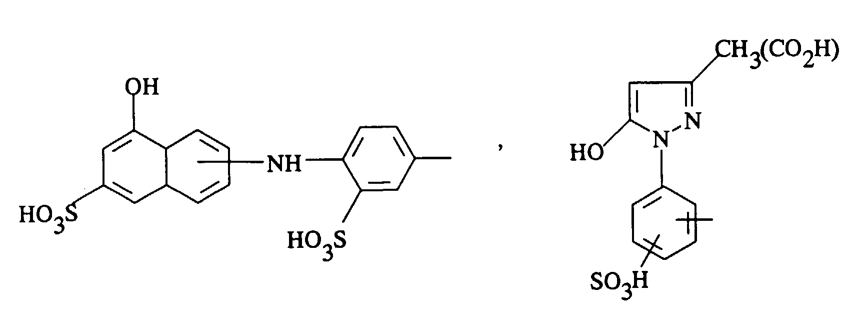

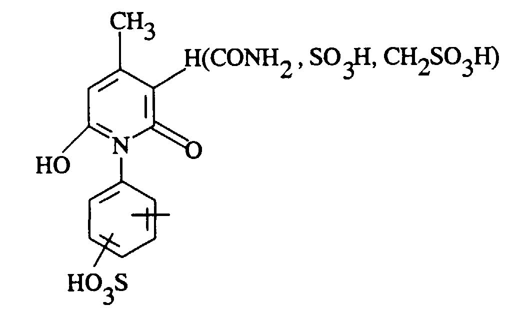

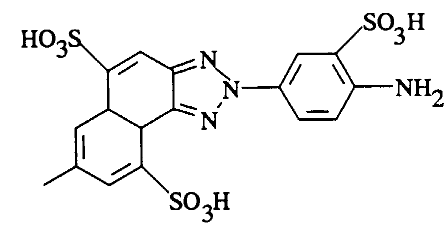

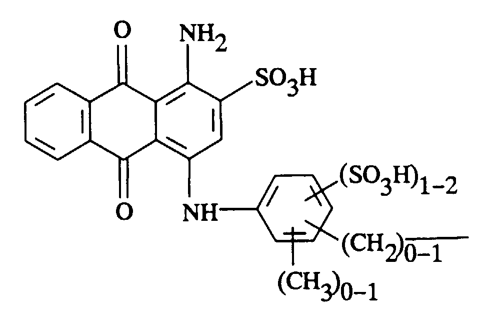

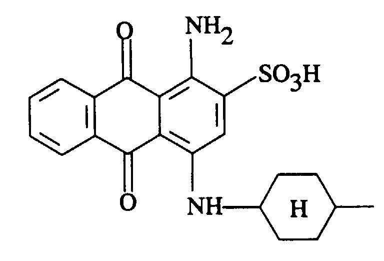

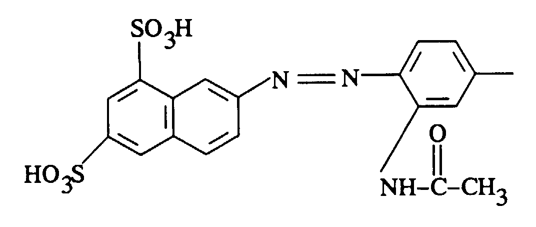

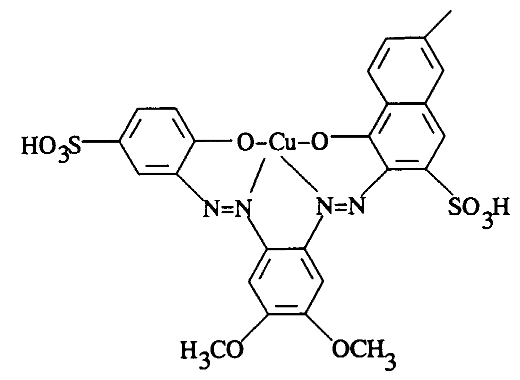

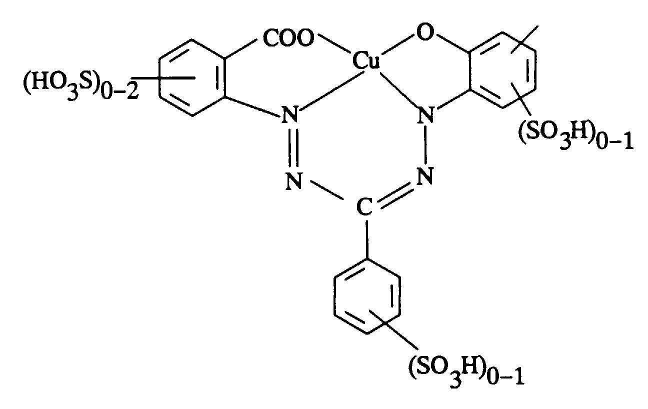

7. Procédé selon au moins l'une des revendications précédentes, caractérisé en ce que l'halogénopyrimidine correspond à au moins l'une des formules suivantes :
